# EUROPEAN PATENT APPLICATION

(11) **EP 3 738 500 A1**
(43) Date of publication of application: **18.11.2020**
(21) Application number: 19174087.7
(22) Date of filing: 13.05.2019
(51) Int. Cl.: A61B 3/12, A61B 3/10, G01N 21/47

(54) **CONFOCAL AND MULTI-SCATTER OPHTHALMOSCOPE**

(71) Applicant: Nederlandse Organisatie voor toegepast- natuurwetenschappelijk Onderzoek TNO, 2595 DA 's-Gravenhage (NL)
(72) Inventor: Schottner, Michael Hardy, 2595 DA 's-Gravenhage (NL); Amelink, Arjen, 2595 DA 's-Gravenhage (NL); Oderwald, Michiel Peter, 2595 DA 's-Gravenhage (NL)
(74) Representative: V.O.

(57) **Abstract**

An opthalmoscope and method for imaging a retina (R). A light pattern (Ri) of source light (Li) is projected onto a retinal focal plane (Pr) coinciding with the retina (R). Light is measured from the retinal focal plane (Pr) resulting from the source light (Li) of the respective light pattern (Ri) interacting with the retina (R). A first part of the light is received in a first detection channel (Cl) from a first area (Rc) on the retina (R) which first area (Rc) overlaps the respective light pattern (Ri) from which said first part of the light originates. A second part of the light received in a second detection channel (C2) from a second area (Ro) on the retina (R) which second area (Ro) is offset by a lateral distance (dR) with respect to the respective light pattern (Ri) from which said second part of the light originates.

## Description

### TECHNICAL FIELD AND BACKGROUND

The present disclosure relates to an ophthalmoscope and method for imaging a retina, e.g. its structure and optical properties.

For the assessment of a several diseases with an impact on optical properties of cells in the human retina, the in-vivo analysis of multiple scattering in the tissue is an important tool. These diseases can be directly related to the vision system, but can also include diseases of the neuronal system like Alzheimer or Parkinson. To obtain additional information, various types of measurements can be performed, e.g. confocal or multi-scatter. However it can be difficult to align the different measurements, e.g. due to artefacts from eye movements.

There is yet a need for an improved ophthalmoscope which can quickly and accurately obtain desired information from retinal measurements.

### SUMMARY

Aspects of the present disclosure relate to an ophthalmoscope for imaging the structure and optical properties of a retina. A light pattern, e.g. focal spot, is projected onto the retina. A first spatial filter is disposed in a conjugate focal plane and configured to pass a first part of the light received from an area on the retina which overlaps said light pattern. This light may be used for confocal imaging of the retina. A second spatial filter is configured to pass a second part of the light from another area on the retina which is offset by a lateral distance with respect to the light pattern, e.g. a ring around the focal spot. In other words, this second part of the light is received from an area which was not directly illuminated. This light may be used to provide additional information on surrounding tissue, e.g. the light scattering and absorbing properties of a tissue volume related to the second spatial filter. By combining the measurements in a single device, a more complete image may be obtained with the benefits of (high spatial resolution) confocal imaging aligned with (lower spatial resolution) determination of other or further optical properties (scattering coefficient, scattering phase function, absorption coefficient).

In preferred embodiments, a single element detector is used to record a respective signal. Such detectors may have superior acquisition speed, e.g. compared to devices with a larger number of detection elements (pixels). Accordingly, problems associated with eye movement may be avoided. Preferably, the second filter is configured to allow accurate determination of the exact distance the light has travelled through the tissue before being retransmitted from the retina and collected. For example, a confocal laser scanning system can be combined with a ringshaped light aperture that can be in the illumination or the detection path. In both cases the same scanning system can be used by both, the illumination and the detection path.

In one embodiment, with a ring shaped aperture is in the illumination system, a second, confocal light source with a different wavelength can be combined using a mirror with a hole, or another beam splitting device based on polarization, or wavelength. In the detection path the two wavelengths can be separated by another beam splitter based on wavelength. For the detection then there can be two confocal detectors at conjugated position. The size of the detector for the ring illumination may have a different aperture/pinhole for the detection. The advantage of this solution is the simplicity in the detection path consisting in two confocal detectors. One can see the light from the center of the scanning, the other from the circular area around this central point.

Another or further embodiment may use a setup with a ring-shape aperture in the detection path. The illumination can be the same as in a standard confocal laser scanning system. In this case the system can work with a single wavelength. The beam splitting can be done spatially by a mirror with a pinhole for the confocal signal in the center or an active mirror with programmable mirror elements.

A preferred embodiment may comprise a combination of a pinhole with a mirror around it and an active mirror. In this case the pinhole defines the light for the confocal part and the active mirror in a conjugated plane to the pinhole can control the dimensions of the ring out of the reflected light that should hit the second sensor. The programmable mirror can be also replaced by a transparent programmable screen like a LCD in the conjugated plane. In any case the lateral distance range from the center of the laser beam in the retina that is used for the second detector can be chosen by the geometrics of the inner and outer diameter of the aperture ring. For the case with the programmable mirror, it is also possible to toggle between two or more settings during the scanning - for every single position, for every line or a full frame. The ring shaped apertures can also be replaced by other shapes that allow to select light from a defined distance from the central confocal laser path.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1A illustrates a side view of light (Li) illuminating a retina (R) in accordance with an illumination pattern (Ri);
FIG 1B illustrates a top view of the retinal focal plane (Pr).
FIGs 2-7 schematically illustrate various embodiments of an opthalmoscope (top) with corresponding representations of the corresponding focal planes (bottom).

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

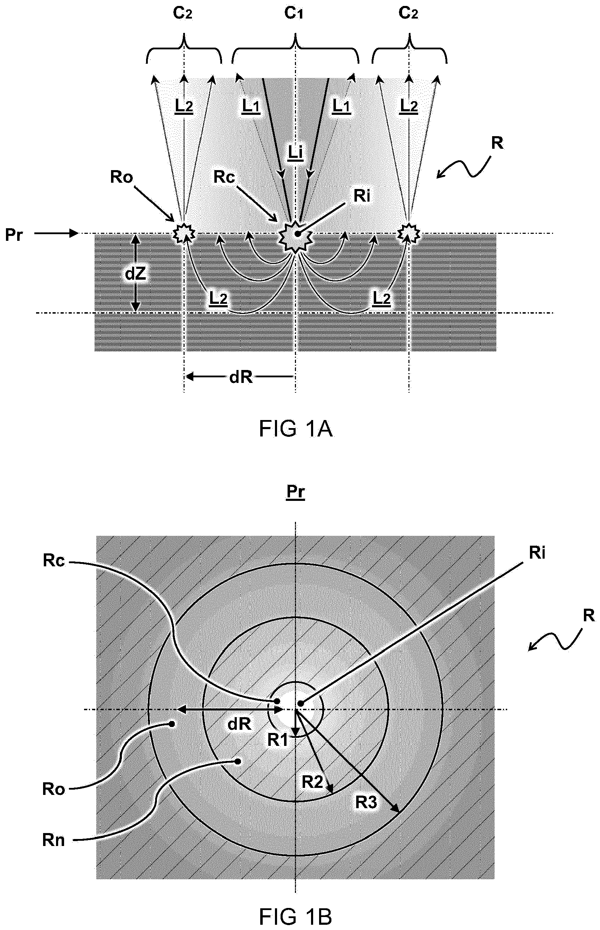

FIG 1A illustrates a side view of light Li illuminating a retina R in accordance with an illumination pattern Ri;

In some embodiments, different part of the light are received and/or reflected along different light paths L1,L2, e.g. following at least partially distinct spatial trajectories. Preferably, the different light paths L1,L2 are recorded in distinct detection channels C1,C2, e.g. by different detectors (not shown here). In a preferred embodiment, all light corresponding to a respective detection channel C1,C2 is measured by a respective single element detector. It will be appreciated that a single element detector may typically be faster than a multi-pixel array and all light hitting the single detection element may be integrated.

In some embodiments, the different light paths L1,L2 are reflected from different areas Rc, Ro of the retina R. In one embodiment, e.g. as shown, the different light paths L1,L2 both originate from the same light pattern Ri. Alternatively, it also may be envisaged that the different light paths L1,L2 are reflected from the same area on the retina, e.g. originating from different illumination patterns (not shown here).

In a preferred embodiment, a first part of the light following the first light path L1 is directly reflected from a first area Rc illuminated by the light pattern Ri. In another or further preferred embodiment, a second part following the second light path L2 is reflected via (multiple) scattering. For example, the second part may be laterally displaced away from the area of the retina R receiving the respective light pattern Ri which produces the scattered light.

In some embodiments, light entering the first detection channel C1 is used to perform a high resolution confocal measurement of the retinal surface while the second detection channel C2 is used to measure other or further properties of surrounding retinal tissue, e.g. the optical transport coefficients (scattering coefficient, scattering phase function and absorption coefficient). The tissue volume over which these properties are averaged depends on the selected radial distance dR, or range of radial distances. Typically, light which is recorded from a second areas Ro at a relatively large distance dR away from the originating light pattern Ri, may travel at relatively large depth dZ through the tissue of the retina R and the optical properties derived from such a measurement are average values of large retinal tissue volumes. Conversely, light received from the illuminated area, or in close proximity to this area, may correspond (mostly) to relatively small sampling volumes. By combining the measurements of the two or more detection channels C1,C2 a more informative lower spatial resolution optical property analysis of the retina R may be obtained without sacrificing the spatial resolution of the original confocal retinal image. For example, the information can be used to construct an image of the retina surface which is augmented by information of concentrations of absorbing molecules such as oxyhemoglobin, deoxyhemoglobin and carotenoids which can be obtained from a (multi-color) measurement of the absorption coefficient of the retinal tissue.

FIG 1B illustrates a top view of the retinal focal plane Pr. In the figure, a number of radial distances R1,R2,R3 are indicated which can be used to describe preferred measures between first and second areas Rc,Ro on the retina R from which light can be recorded in different detection channels C1,C2. For example, an outer spot radius R1 corresponds to a maximum distance between a center of the light pattern Ri and an edge of the first area Rc from which light enters the first detection channel C1. For example, a inner ring radius R2 corresponds to a minimal distance between the center of the light pattern Ri and (closest) edge of the first area second area Ro from which light enters the second detection channel C2. For example, a outer ring radius R3 corresponds to a maximum distance between the center of the light pattern Ri and (furthest) edge of the second area Ro from which light enters the second detection channel C2. Of course also other corresponding distances may be used depending on the shape of the light pattern Ri.

In some embodiments, e.g. as illustrated here, the light pattern Ri is formed by a (central) focal spot. In another or further embodiment, the light pattern Ri may have other forms, e.g. ring shaped or another pattern having a (radial) offset from an imaged location. Typically, the focal spot or other light pattern may be scanned over the retina R to form a spatially resolved image by combining measurements at different locations.

In some embodiments, light originating from a first area Rc including or coinciding with the light pattern Ri (e.g. focal spot or other pattern) is recorded in a first detection channel C1. For example, the light from the retina is imaged by projection optics onto a first spatial filter (not shown here). For example, the first spatial filter comprises a pinhole which exclusively passes light from the focal spot to a respective light detector, at least does not pass light from a second area Ro which is noncontiguous with the first area Rc. In this way a confocal image of the retina may be recorded. Also other corresponding shapes of the light pattern and spatial filter may be envisaged.

In some embodiments, light originating from a second area Ro which does not include the focal spot or other light pattern Ri, is recorded in a second detection channel C2. For example, the second area Ro is offset at a radial distance dR from the light pattern Ri, as illustrated. In some embodiments, light from the retina R is imaged on a second spatial filter (not shown here). For example, the second spatial filter comprises a ring shape or other transmissive or reflecting area having an offset to exclusively pass light from the second area Ro to a respective light detector, at least not pass light directly originating from the first area Rc, e.g. focal spot.

In some embodiments, light entering the first detection channel C1 originates exclusively from within the outer spot radius R1 (or other distance) overlapping the light pattern Ri, e.g. with respect to a center of the focal spot. Preferably, the outer spot radius R1 is selected to substantially coincide with the light pattern Ri, e.g. having the same or similar radius as the focal spot to within ±100%, ±50%, ±20%, or less. The closer, the recorded area matches the light pattern Ri, the more efficient the measurement while avoiding undesired (diffuse) light.

In some embodiments, light entering the second detection channel C2 originates from a specific distance dR (or range of distances) separated from the center or edge of the light pattern Ri. For example, in the embodiment shown, the second area Ro may be formed by an area of the retina which is more than the inner ring radius R2 (or other distance) away from the center or edge of the light pattern Ri e.g. focal spot, and less than the outer ring radius R3 (or other distance).

In a preferred embodiment, the inner ring radius R2 is larger than the outer spot radius R1. In other words, the second area Ro is preferably noncontiguous or disjoint from the first area Rc. Accordingly, there can be a non-imaged area Rn on the retina separating the second area Ro from the first area Rc. For example, the hatched areas in the figure indicate areas of the retina from which light may be blocked to reach any detector, e.g. neither the first nor the second detection channel. For example, the light is blocked by one or more spatial filters in a respective light path. For example, a spatial filter comprises an absorptive coating in a corresponding area between the outer spot radius R1 and inner ring radius R2. Accordingly, light entering the second channel C2 has preferably traveled at least some minimum distance (R2 - R1) from the point of illumination through the tissue, but no more than the maximum distance (R3 - R1).

In some embodiments, the minimum distance (R2 - R1) between the non-contiguous areas Ro and Rc imaged on respective spatial filters is more than ten micrometer, more than fifty micrometer, more than hundred micrometer, more than a few hundred micrometers, more than one millimeter, or more. In other or further embodiments, the maximum distance (R3 - R1) between the second area Ro and the first area Rc is less than two millimeter, less than one millimeter, less than five hundred micrometer, less than two hundred micrometer, or less. These minimum and maximum distances may correspond to typically desired photon path lengths for probing the retinal tissue. Radial distances may also be determined in relative proportion. In a preferred embodiment, the inner ring radius R2 is larger than the outer spot radius R1 by at least a factor 1.1 (ten percent), 1.5 (fifty percent), 2 (two hundred percent), 3 (three hundred percent), 5 (five hundred percent), or more.

In some embodiments, a difference between the outer ring radius R3 and inner ring radius R2 is relatively small. In other words, the range of radial distances dR entering the second detection channel C2 is relatively narrow. For example, R3 is larger than R2 by at least a factor 1.01, 1.1, or 1.5, but less than a factor two or three. Alternatively, or additionally, the thickness of the ring (R3 - R2) may be compared relative to a distance (R2 - R1) between the ring and the illuminated area, e.g. (R2 - R1) ≥ N * (R3 - R2) where the factor N is at least half, preferably at least one, or even more than two. These or other measures may ensure, light entering the second channel C2 originates from a well defined yet sufficiently broad band of radial distances dR and can be correlated with corresponding path lengths.

It will be understood that the absolute distances dR on the retina may be related to corresponding, but possibly different distances dR' on the respective spatial filter, depending on the magnification of the projection system. On the other hand, relative distances may be the same or similar for features on the corresponding spatial filters (at least assuming the same magnification is used for different filters).

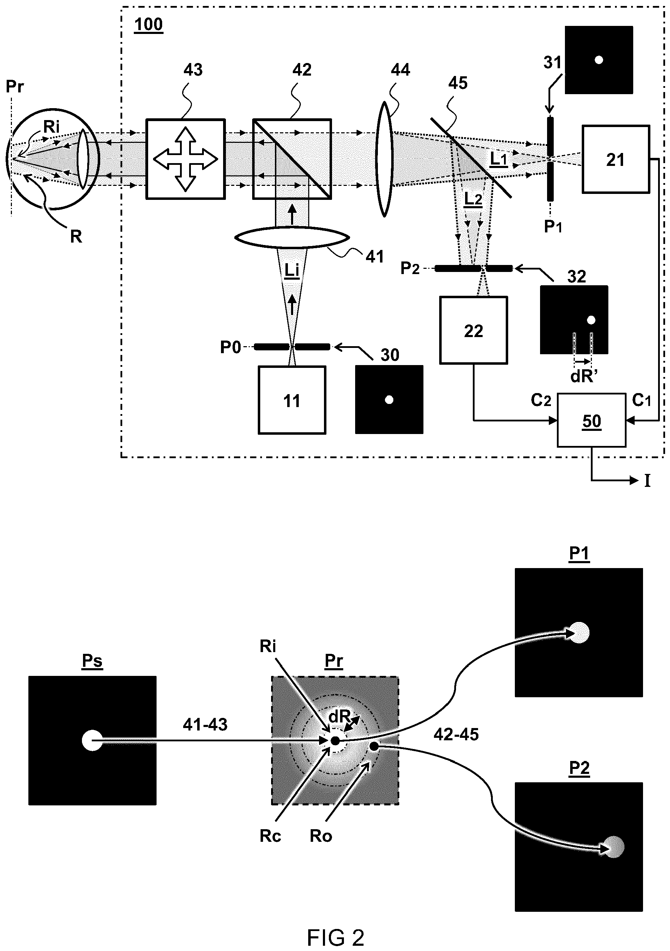

FIGs 2-7 (top parts) illustrate respective embodiments of an opthalmoscope 100 for imaging a retina R. For example, the ophthalmoscope 100 is a scanning laser opthalmoscope. The bottom parts of the figures show corresponding representations of the relevant focal planes. In the following, various aspects are described with reference to the embodiments. Of course, it will be clear that the features described with reference to one or more embodiments may also be combined or substituted with corresponding features from other or further embodiments.

Some embodiments for imaging the retina R comprise projecting a respective light pattern Ri of source light Li from onto a retinal focal plane Pr coinciding with the retina R. Other or further embodiments comprise measuring light from the retinal focal plane Pr resulting from the source light Li of the respective light pattern Ri interacting with the retina R. Other or further embodiments comprise measuring a first part of the light received in a first detection channel C1 from a first area Rc on the retina R which first area Rc overlaps the respective light pattern Ri from which said first part of the light originates. Other or further embodiments comprise measuring a second part of the light received in a second detection channel C2 of the respective light detector 22 from a second area Ro on the retina R which second area Ro is offset by a lateral distance dR with respect to the respective light pattern Ri from which said second part of the light originates. Preferably, the first and second parts of the light are measured simultaneously.

In some embodiments, the measurements of the first and second detection channels C1,C2 are combined to form one or more overlapping images I of the retina R. In other or further embodiments, a position of the light pattern Ri is scanned over the retina R, wherein the measurements of the first and second detection channels C1,C2 at each position are combined to form a pixel of the image I at that position. In other or further embodiments, respective overlapping images of the retina surface and retinal optical properties are constructed based on the respective detection channels C1,C2, wherein pixels are aligned across the images based on a corresponding position of the same light pattern Ri used for measuring those pixels. In other or further embodiments, an image of tissue optical properties of the retina is calculated based on respective measurements in the second detection channel C2, wherein the averaged tissue volume is controlled by setting a radial distance dR between a center of the light pattern Ri and the second area Ro from which light is received in the second detection channel C2.

In some embodiments, the ophthalmoscope 100 comprises an illumination system. Preferably, the illumination system comprises at least one light source 11,12, e.g. laser. In some embodiments, e.g. as shown, a first set of projection optics 41-43 is configured to project a respective light pattern Ri of source light Li from the respective light source 11,12 onto a retinal focal plane Pr coinciding with the retina R.

In some embodiments, the ophthalmoscope 100 comprises a measurement system. Preferably, the measurement system comprises at one or more light detectors 21,22. In some embodiments, e.g. as shown, a second set of projection optics 42-45 is configured to measure light from the retinal focal plane Pr. The measured light results from the source light Li of the respective light pattern Ri interacting with the retina R.

In some embodiments, the ophthalmoscope 100 comprises a first spatial filter 31 disposed in a first light path L1 between a respective pair 11,21 of the at least one light source 11 and at least one light detector 21,22. Preferably, the first spatial filter 31 is configured to predominantly or exclusively pass a first part of the light received in a first detection channel C1 of the respective detector 21 from a first area Rc on the retina R. For example, the first area Rc overlaps the respective light pattern Ri from which said first part of the light originates.

In some embodiments, the ophthalmoscope 100 comprises a second spatial filter 32, disposed in the second light path L2 between a respective pair 11,22 of the at least one light source 11 and at least one light detector 21,22. Preferably, the second spatial filter 32 is configured to predominantly or exclusively pass a second part of the light received in a second detection channel C2 of the respective light detector 22 from a second area Ro on the retina R. For example, the second area Ro is offset by a lateral distance dR with respect to the respective light pattern Ri from which said second part of the light originates.

In some embodiments, the projection optics 40-45 comprise one or more lenses and/or (curved) mirrors to form the light paths L1,L2; and project or image respective light patterns between the retinal focal plane Pr and its conjugate focal planes P0,P1,P2, e.g. planes where an image of the retina can be formed by the intermediate optics. In a preferred embodiment, the first and second spatial filters 31 are disposed in one or more of the conjugate focal planes designated as P1 and/or P2. In some embodiments, a third spatial filter 30 is arranged at another conjugate focal plane designated as P0.

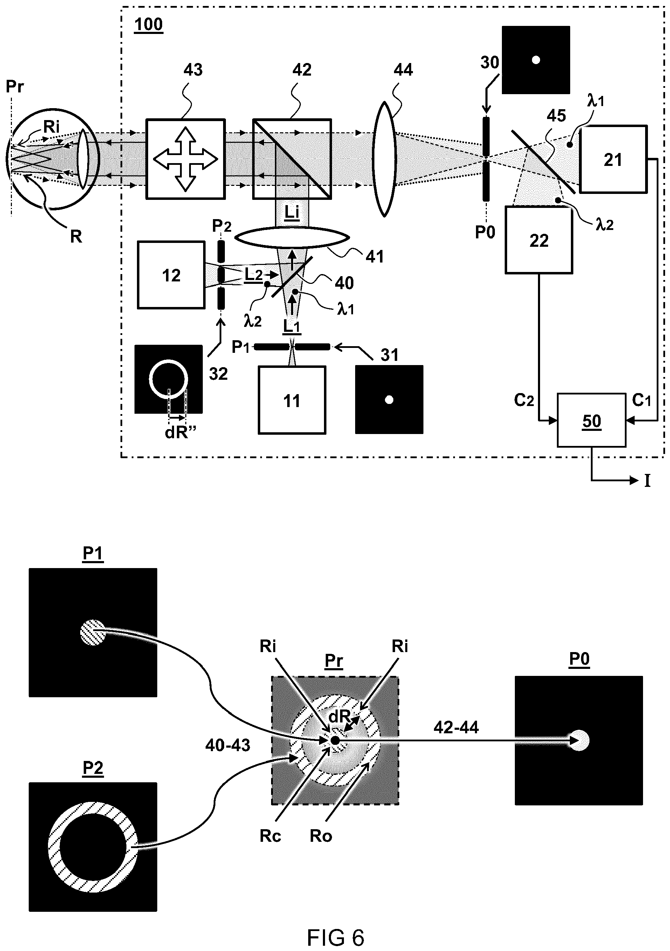

In some embodiments, the first set of projection optics 40-43 is configured to project an image of one or more patterns arranged at an object plane onto the retinal focal plane Pr. In some embodiments, e.g. as shown in FIGs 2-5, a shape of the projected light pattern Ri is determined by the third spatial filter 30 arranged between the light source 11 and the retina R. In other or further embodiments, e.g. as shown in FIGs 6-7, a shape of the projected one or more light patterns Ri is determined by the first and/or second spatial filters 31,32. Alternatively to a separate spatial filter 30 it can also be envisaged that a shape of the respective light source 31,32 directly determines a shape of the projected pattern.

In some embodiments, the second set of projection optics 42-45 is configured to project an image the retinal focal plane Pr onto a respective one or more image planes. Preferably, the image is projected on a respective spatial filter which is configured to pass (transmit or reflect) only the intended part of the image to a respective detector.

In some embodiments, one or more of the optical components 42,43 may be shared between the first and second sets of projection optics. In the embodiments shown, a beam splitter/combiner 42 acts as a reflector in the first set while acting as a transmitter in the second set. Of course this may also be reversed.

In some embodiments, beam steering optics 43 are configured to controllably vary a position of the light pattern Ri on the retina. For example, an illuminated spot may be scanned over the surface of the retina. In the embodiments shown, the steering optics 43 are arranged to both transmit and receive light to/from the retina. For example, the beam steering optics 43 comprise one or more controllable mirrors. Also other ways of controlling the position of the light pattern can be envisaged, e.g. moving the ophtalmoscope and/or retina.

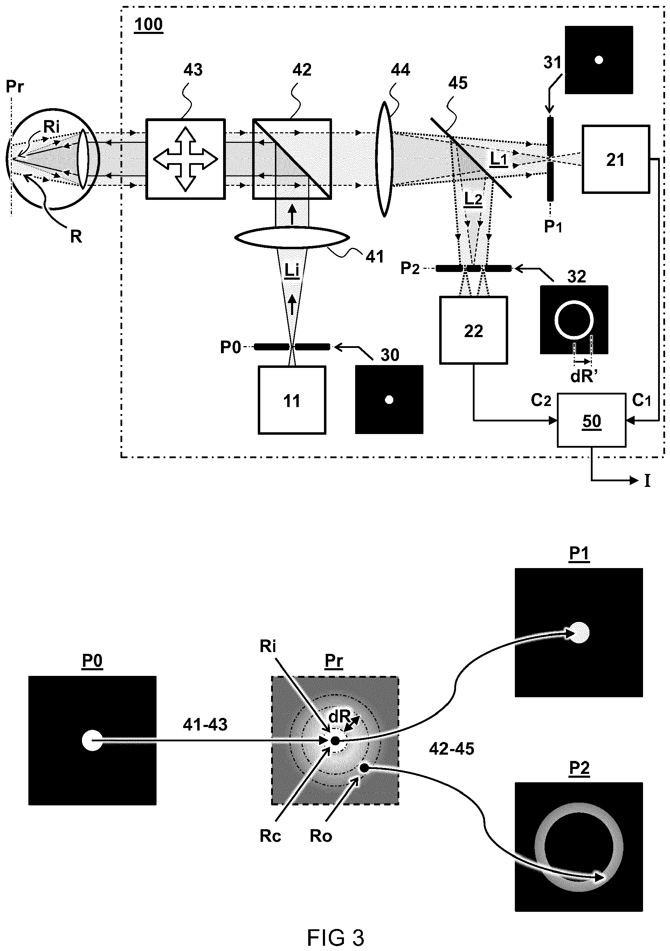

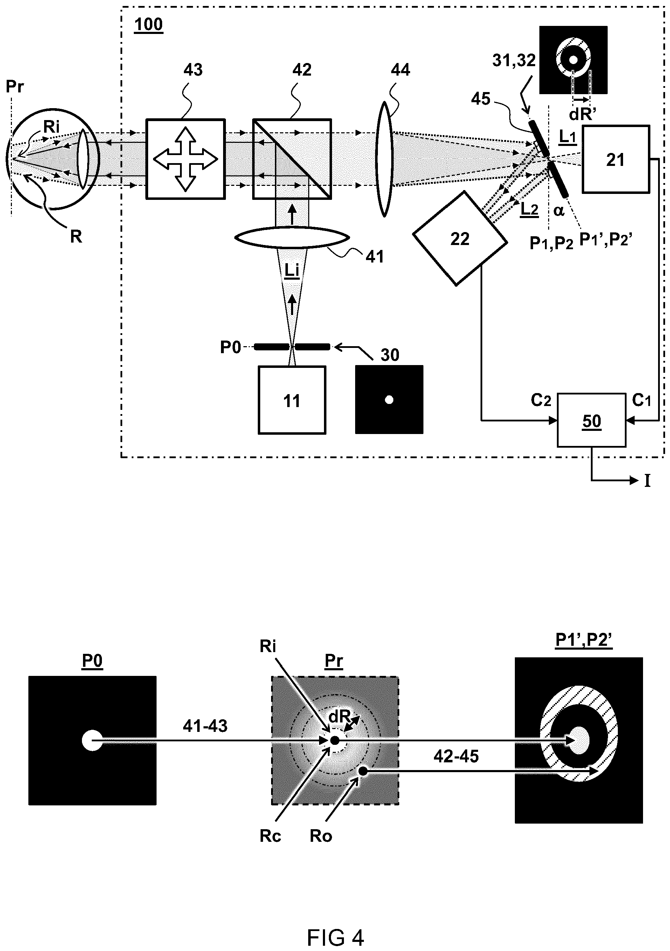

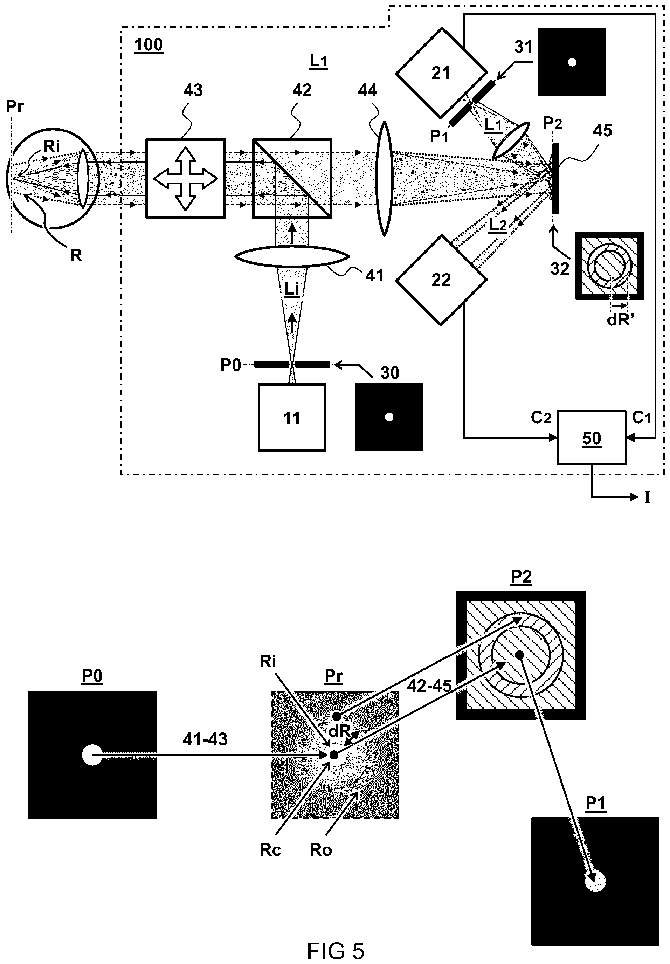

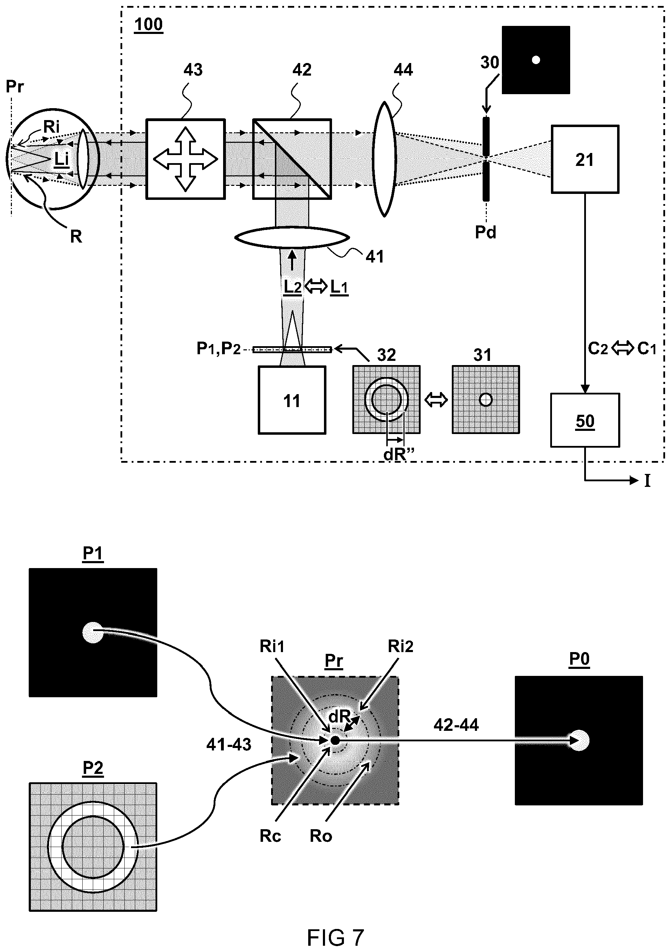

In some embodiments, e.g. shown in FIGs 2 and 3, the second set of projection optics is configured to split the beam for projecting duplicate images on different spatial filters 31,32. Each filter may then pass a respective part of the image to the detector. In other or further embodiments, e.g. as shown in FIGs 4 and 5, the second set of projection optics is configured to project one image which is split in different components by an element combining multiple of the spatial filters, e.g. a pinhole with a surrounding ring shaped mirror as shown in FIG 4, or mirror elements splitting the light in different directions as shown in FIG 5. In other or further embodiments, e.g. shown in FIGs 6 and 7, the second set of projection optics is configured to project the image onto a single spatial filter 30 such as a pinhole, e.g. when the light pattern Ri projected on the retina is varied. While it is preferred to have a separate spatial filter between the retina R and respective detector for better control of the spatial profile to be imaged, it may also be envisaged that a shape of the detection element is adapted to receive only the intended light.

In some embodiments, e.g. as shown in FIGs 2-5, the first and second spatial filters 31, are disposed in respective image planes. In other or further embodiments, the light pattern Ri is a focal spot. Preferably, a diameter of the focal spot is relatively small, e.g. less than hundred micrometer, preferably less than fifty micrometer, less than ten micrometer, less than one micrometer, or even less, e.g. diffraction limited.

In some embodiments, a part of the first light path L1 overlaps a part of the second light path L2. In other words the first light path L1 and second light path L2 may travel together at least part of the way. This may be advantageous, e.g. in that at least some of the optical components (41-44), may be used to form both light paths L1,L2.In some embodiments, at least some of the first light path L1 is distinct from the second light path L2. In other words the first light path L1 may not completely overlap the second light path L2. This may be advantageous, e.g. in that the light can be well separated, e.g. in the detection. For example, it may be prevented that the first part of the light of the first light path L1 interferes with the second part of the light in the second light path L2, at least along the distinct path. Alternatively, or additionally, it may be envisaged that different light patterns are alternated or modulated, e.g. as shown in FIG 7. Still, at least the part of the trajectory through the retina (direct reflection or multiple-scattering) may be different depending on the light pattern.

In a preferred embodiment, e.g. as shown in FIGs 2-6, the first part of the light is measured in the first detection channel C1 of a first light detector 21, and the second part of the light is measured in the second detection channel C2 of a separate second light detector 22.This may be advantageous, e.g. in that the light of different parts is well separated for the measurement. Furthermore, a pair of relatively simple light detectors may be used to measure all light from the respective light paths L1,L2 after being filtered by the respective spatial filter.

In another or further preferred embodiment, each of the separate light detectors 21,22 are single element detectors. Accordingly, all light entering a respective detection channel is measured by a respective single detection element. For example, when using two detection channels C1,C2 the detection system may be formed by only two single detector elements. For example, the single element detectors may be formed by two photodiodes. Alternatively, or additionally, the system may alternate between different types of measurements, e.g. as shown in FIG 7. Alternatively, or additionally, it may be envisaged to measure both parts of the light by the same light detector (not shown). For example, the first part may be projected on a first set of pixels and the second part may be projected on a distinct second set of pixels.

Also more than two detection channels may be envisaged, e.g. an additional third or fourth detection channel may be associated with other respective radial distances from the light pattern Ri. By measuring two, three, four, or more different radial distances in two or more separate detection channels, an even more complete analysis of the retinal tissue may be obtained. In some embodiments, a radial distance of light entering the second detection channel C2 may be varied while taking confocal measurements in the first detection channel C1. For example, a position of the second spatial filter 32 and/or shape of the projected light pattern Ri can be varied.

In some embodiments, e.g. as shown in FIGs 2-6, a beam splitter 45 is arranged to split a common part of the light path into distinct parts of the first light path L1 and second light path L2. For example, the first light path L1 may lead to the first detection channel C1 and the second light path L2 may lead to the second detection channel C2. In other or further embodiments, e.g. as shown in FIGs 1 and 2, the first spatial filter 31 is disposed in the first light path L1 between the beam splitter 45 and the light detector 21,22; and the second spatial filter 32 is disposed in the second light path L2 between the beam splitter 45 and the second light detector 21,22; and the second spatial filter 32

In some embodiments, e.g. as shown in FIGs 2 and 3, the beam splitter 45 may simply reflect a specific percentage of all light, e.g. an achromatic and/or non-polarizing beam splitter. Because the first part of the light which originate from a direct illuminated region may typically be more intense than the second part of the light which does not originate from a direct illuminated region, the beam splitter may be selected to reflect more of the less intense second part. For example, the beam splitter may split more than fifty percent of all light to second detection channel C2, more than sixty percent, more than seventy percent, more than eighty percent, more than ninety percent, or more.

In some embodiments, e.g. as shown in FIG 2, the ophthalmoscope 100 comprises one light source 11 and two light detectors 21,22. The first and second light paths L1,L2 are split by a beam splitter 45. The first spatial filter 31 is arranged in the first light path L1 in a first conjugate focal plane P1 between the retina R and the first light detector 21. The second spatial filter 32 is arranged in the second light path L2 in a second conjugate focal plane P2 between the retina R and the second light detector 22. A third spatial filter 30 is disposed between the light source 11 and the light pattern Ri to shape the light pattern Ri. The spatial filter 30 is formed by a pinhole to form the light pattern Ri as a focal spot on the retina R. The first spatial filter 31 is formed by another pinhole which is confocal with the focal spot on the retina R. The second spatial filter 32 is formed by yet another pinhole which is not confocal with respect the focal spot illuminating the retina R. In other words, the second spatial filter 32 is configured to block the light from the focal spot and only pass light having a projected radial offset dR'.

In some embodiments, e.g. as shown in FIG 3, the second spatial filter 32 has a ring shape instead of a pinhole which is confocal with the focal spot on the retina R. This can have the advantage of more light being collected but originating from the same or similar radial offset from the point of illumination.

In some embodiments, e.g. as shown in FIGs 4 and 5, the beam splitter 45 may itself form one or more of the spatial filters 31,32. For example, the beam splitter 45 is configured to pass the different parts of the light in different directions. In other or further embodiments, the first and second spatial filters 31,32 are formed on a beam splitter 45. For example, the beam splitter 45 is arranged at or near an imaging or focal plane P1,P2 of the second set of projection optics 42-45.

In some embodiments, e.g. as shown in FIG 4, the first spatial filter 31 is formed by a pinhole through the beam splitter 45 and the second spatial filter 32 is formed by a reflective ring around the pinhole. For example, the pinhole is configured to pass a confocal spot of illuminating the retina R to the first detector 21. For example, the reflective ring is configured to pass (reflect) a ring shaped area image of the retina around the focal spot to the light detector 21,22. This can have the advantage of more efficient light collection, since the beam is not first split and all intended light can hit the respective spatial filters.

Preferably, the beam splitting spatial filters are placed at a relatively small angle α with respect to the conjugate focal planes, e.g. less than thirty degrees (plane angle), preferably less than twenty or even less than ten degrees. Alternatively, or additionally, the image may be projected with a relatively large depth of focus, e.g. wherein the depth of focus is larger than a maximum difference in axial positions of the tilted spatial filter along the optical axis. In one embodiment, e.g. as shown, a shape of one or more of the spatial filters 31,32 is adapted to the angle α at which it is placed with respect to the conjugate focal planes. For example, the ring shape may be extended to form an ellipse, e.g. stretched inversely proportional to a cosine of the angle α. The ellipse may be asymmetric, e.g. to match a converging beam in which it is placed. Optionally, also the pinhole may be stretched and/or drilled through the plate at a corresponding angle α.

In some embodiments, a position of the light being passed by one or more of the spatial filters 31,32 to a respective detector 21,22 is controllable, e.g. determined by a controller [not shown] and/or program on the controller. For example, a position of a pinhole forming the second spatial filter 32, e.g. as shown in FIG 2, may be varied by the controller. For example, a number of measurements using the same position of a focal spot on the retina can be used while the projected radial distance dR' passed through the second spatial filter 32 is varied. In other or further embodiments, e.g. as shown in FIG 5 and 7, one or more of the spatial filters 31,32 is formed by a digital light projector (DLP). For example, a pattern of the DLP may be set by a controller [not shown].

In some embodiments, e.g. as shown in FIG 5, at least one of the spatial filters 32 is formed by an active (DLP) mirror. Such device may have programmable mirror elements and is also referred to as a digital mirror device (DMD). In some embodiments, the second spatial filter 32 is formed by selectively activated (or non-activated) mirror elements of a digital mirror device (DMD) forming a beam splitter 45. The selected mirror elements may reflect the second part of the light along the second light path L2 to the second light detector 22. The remaining light is reflected in another direction. This remaining light is re-imaged onto the first spatial filter 31 before reaching the first light detector 21. In this way, only light corresponding to the focal spot forming the light pattern Ri is recorded in the first detection channel C1. In some embodiments, a shape of the second spatial filter 32 is determined by a controller activating specific mirror elements. In other or further embodiments, the shape is varied over time to record different signals. For example, the controller is configured to produce ring shapes having variable radial distances dR' with respect to the projected focal spot. In this way a signal as a function of radial distance can be recorded, e.g. used for constructing an image of the optical properties of the retina with higher accuracy.

In some embodiments, e.g. as shown in FIG 5, the DLP is configured to pass a first light pattern in one direction and a second light pattern in another direction. In one embodiment, e.g. as shown, a ring shaped pattern is reflected towards the second light detector 22 while the central spot is reflected towards the first light detector 21. In some embodiments, e.g. as shown, the first spatial filter 31 is arranged in a further focal plane P1 between the DLP and the first light detector. In this way extraneous reflected light (not coming from the focal spot) may be filtered out.

In some embodiments, e.g. as shown in FIG 6, the beam splitter 45 is chromatic, e.g. configured to reflect or pass a first range of wavelengths while passing or reflecting, respectively, another range of wavelengths (distinct from the first range). In some embodiments, a spectrum of light reflected from the retina may depend on whether the light originated from a directly illuminated part or from a non-illuminated part. In other or further embodiments, the ophthalmoscope 100 comprises at least two light sources 11,12 having different wavelengths λ1,λ2. In another or further embodiment, the ophthalmoscope 100 comprises a beam combiner 46 to combine the different wavelength light from the light sources 11,12 on a common part of the respective light paths L1,L2. In some embodiments, the first spatial filter 31 is placed in the first light path L1 between the first light source 11 and the beam combiner 46; and the second spatial filter 32 is placed in the second light path L2 between the second light source 12 and the beam combiner 46.

In some embodiments, e.g. as shown in FIG 7, one or more spatial filters 31,32 are formed by a transmissive DLP screen, e.g. LCD or other device. In the embodiment shown, the DLP screen is placed between the light source 11 and the retina R. In another or further embodiment (not shown) a DLP screen is placed between the retina R and one or more light detectors. For example, the DLP mirror in FIG 5 may be replaced by a transmissive DLP screen. In some embodiments, a controllable spatial filter such as a DLP is used to modulate the light pattern Ri projected on the retina R. For example, the pattern is modulated between a focal spot and one or more ring shapes, optionally having variable size. Alternatively, or in addition, also the image passed to the detector may be varied by a DLP (not shown here). The light detection may be synchronized, e.g. alternated or phase locked, to the variable light pattern to record the different signals in separate detection channels C1,C2. The processor 50 may then reconstruct an image I or other signal based on the combined measurements.

For the purpose of clarity and a concise description, features are described herein as part of the same or separate embodiments, however, it will be appreciated that the scope of the invention may include embodiments having combinations of all or some of the features described. For example, while embodiments were shown for simultaneously performing confocal and scattering measurements using a set op spatial filters, also alternative ways may be envisaged by those skilled in the art having the benefit of the present disclosure for achieving a similar function and result. E.g. optical components may be combined or split up into one or more alternative components. The various elements of the embodiments as discussed and shown offer certain advantages, such as quick and reliable measurement of the retinal surface and deeper tissue. Of course, it is to be appreciated that any one of the above embodiments or processes may be combined with one or more other embodiments or processes to provide even further improvements in finding and matching designs and advantages. It is appreciated that this disclosure offers particular advantages to retinal measurements, and in general can be applied also for contactless measurement of other tissue.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. An ophthalmoscope (100) for imaging a retina (R), the ophthalmoscope (100) comprising
- an illumination system with at least one light source (11,12) and a first set of projection optics (41-43) configured to project a respective light pattern (Ri) of source light (Li) from the respective light source (11,12) onto a retinal focal plane (Pr) coinciding with the retina (R);
- a measurement system with at least one light detector (21,22) and a second set of projection optics (42-45) configured to measure light from the retinal focal plane (Pr) resulting from the source light (Li) of the respective light pattern (Ri) interacting with the retina (R);
- a first spatial filter (31) disposed in a first light path (L1) between a respective pair (11,21) of the at least one light source (11) and at least one light detector (21,22), wherein the first spatial filter (31) is configured to exclusively pass a first part of the light received in a first detection channel (C1) of the respective detector (21) from a first area (Rc) on the retina (R) which first area (Rc) overlaps the respective light pattern (Ri) from which said first part of the light originates;
- a second spatial filter (32), disposed in the second light path (L2) between a respective pair (11,22) of the at least one light source (11) and at least one light detector (21,22), wherein the second spatial filter (32) is configured to exclusively pass a second part of the light received in a second detection channel (C2) of the respective light detector (22) from a second area (Ro) on the retina (R) which second area (Ro) is offset by a lateral distance (dR) with respect to the respective light pattern (Ri) from which said second part of the light originates.

2. The ophthalmoscope (100) according to claim 1, wherein at least some of the first light path (L1) is distinct from the second light path (L2), wherein the first part of the light is measured in the first detection channel (C1) of a first light detector (21), and the second part of the light is measured in the second detection channel (C2) of a separate second light detector (22).

3. The ophthalmoscope (100) according to any of the preceding claims, wherein the light detectors (21,22) are single element detectors to receive all light on the respective detector in the respective single detection channel (C1,C2).

4. The ophthalmoscope (100) according to any of the preceding claims, wherein the light pattern (Ri) is formed by a focal spot on the retina (R), wherein the first area (Rc) has an outer spot radius (R1) from a center of the focal spot to overlap with the light pattern (Ri), wherein the second area (Ro) is formed by a ring around the light pattern (Ri), the ring defined between an inner ring radius (R2) from the center of the focal spot and an outer ring radius (R3) from the center of the focal spot, wherein the inner ring radius (R2) of the second area (Ro) is larger than the outer spot radius (R1) of the first area (Rc) by at least a factor 1.1, wherein the outer ring radius (R3) is larger than the inner ring radius by factor less than two.

5. The ophthalmoscope (100) according to any of the preceding claims, wherein the first spatial filter (31) is formed by a pinhole which is confocal with the light pattern (Ri) formed by a focal spot on the retina (R), wherein the second spatial filter (32) is formed by one or more apertures or mirror elements having a radial offset (dR') from an image of the focal spot.

6. The ophthalmoscope (100) according to any of claims 1-5, wherein the first and second spatial filters (31,32) are formed on a beam splitter (45), wherein the first spatial filter (31) is formed by a pinhole through the beam splitter (45) and the second spatial filter (32) is formed by a reflective ring around the pinhole, with a non reflective ring there between.

7. The ophthalmoscope (100) according to any of claims 1-5, wherein the first and second light paths (L1,L2) are split by a beam splitter (45), wherein the first spatial filter (31) is arranged in the first light path (L1) in a first conjugate focal plane (P1) between the beam splitter (45) and the first light detector (21), and the second spatial filter (32) is arranged in the second light path (L2) in a second conjugate focal plane (P2) between the beam splitter (45) and the second light detector (22).

8. The ophthalmoscope (100) according to any of claims 1-5, comprising at least two light sources (11,12) having different wavelengths (λ1,λ2), wherein the first spatial filter (31) is arranged in the first light path (L1) between a first light source (11) and a beam combiner (46); and the second spatial filter (32) is arranged in the second light path (L2) between the second light source (12) and the beam combiner (46).

9. The ophthalmoscope (100) according to any of the preceding claims, wherein a position of the light being passed by one or more of the spatial filters (31,32) to a respective detector (21,22) is determined by a controller.

10. The ophthalmoscope (100) according to any of the preceding claims, wherein one or more of the spatial filters (31,32) is formed by a digital light projector, wherein a shape or position of an image passing the respective spatial filter (31,32) is determined by controlling the digital light projector.

11. A method for imaging a retina (R), the method comprising
- projecting a respective light pattern (Ri) of source light (Li) from onto a retinal focal plane (Pr) coinciding with the retina (R);
- measuring light from the retinal focal plane (Pr) resulting from the source light (Li) of the respective light pattern (Ri) interacting with the retina (R);
- wherein a first spatial filter (31) is used for passing a first part of the light to be measured in a first detection channel (C1) from a first area (Rc) on the retina (R) which first area (Rc) overlaps the respective light pattern (Ri) from which said first part of the light originates;
- wherein a second spatial filter (32) is used for passing a second part of the light to be measured in a second detection channel (C2) from a second area (Ro) on the retina (R) which second area (Ro) is offset by a lateral distance (dR) with respect to the respective light pattern (Ri) from which said second part of the light originates.

12. The method according to claim 11, wherein the measurements of the first and second detection channels (C1,C2) are combined to form one or more overlapping images (I) of the retina (R).

13. The method according to any of claims 11-12, wherein a position of the light pattern (Ri) is scanned over the retina (R), wherein the measurements of the first and second detection channels (C1,C2) at each position are combined to form a pixel of the image (I) at that position.

14. The method according to any of claims 11-13, wherein respective overlapping images of the retina surface and retina tissue optical properties are constructed based on the respective detection channels (C1,C2), wherein pixels are aligned across the images based on a corresponding position of the same light pattern (Ri) used for measuring those pixels.

15. The method according to any of claims 11-14, wherein an image of optical properties of the retina tissue is calculated based on respective measurements in the second detection channel (C2), wherein a path length and tissue volume over which the optical properties are averaged is controlled by setting a radial distance (dR) between a center of the light pattern (Ri) and the second area (Ro) from which light is received in the second detection channel (C2).
